# EUROPÄISCHE PATENTSCHRIFT

(11) **EP 1 144 620 B1**
(45) Veröffentlichungstag und Bekanntmachung des Hinweises auf die Patenterteilung: **03.09.2003**
(21) Anmeldenummer: 99959300.7
(22) Anmeldetag: 23.11.1999
(51) Int. Cl.: C12N 15/10, H01F 1/00, H01F 1/11, C09C 3/06, C04B 35/628

(54) **MAGNETISCHE PARTIKEL ZUR REINIGUNG VON NUKLEINSÄUREN**
MAGNETIC PARTICLES FOR PURIFYING NUCLEIC ACIDS
PARTICULES MAGNETIQUES POUR PURIFIER DES ACIDES NUCLEIQUES

(30) Priorität: 30.11.1998 DE 19855259; 30.11.1998 DE 19854973
(43) Veröffentlichungstag der Anmeldung: 17.10.2001
(73) Patentinhaber: Roche Diagnostics GmbH, 68298 Mannheim (DE)
(72) Erfinder: HARTTIG, Herbert, D-67122 Altrip (DE); RIEDLING, Michael, D-66386 St. Ingbert (DE); MENNIG, Martin, D-66287 Quierschied (DE); SCHMIDT, Helmut, D-66130 Saarbrücken-Güdingen (DE)
(86) Internationale Anmeldenummer: EP9908996
(87) Internationale Veröffentlichungsnummer: WO00032762

(56) Entgegenhaltungen:
- WO-A-98/31840
- DE-A- 19 520 964
- DE-A- 19 537 985

## Beschreibung

Gegenstand der Anmeldung ist eine Zubereitung von Partikeln mit einer Glasoberfläche, ein Verfahren zur Herstellung einer solchen Zubereitung sowie ein Verfahren zur Reinigung von Nukleinsäuren mit Hilfe dieser Zubereitung.

Nukleinsäuren sind in jüngerer Zeit immer mehr in den Blickpunkt des Interesses der medizinischen Diagnostik gerückt. So wurden mittlerweile eine Vielzahl von Nachweisverfahren erarbeitet, bei denen die Anwesenheit oder Abwesenheit bestimmter Nukleinsäuren als Anzeichen für eine Erkrankung bewertet wird. Hierzu gehören z. B. Nachweise infektiöser Organismen, z. B. von Viren oder Bakterien in Körperflüssigkeiten, aber auch der Nachweis von Mutationen in genomischen Nukleinsäuren, z. B. in der Onkologie. Nukleinsäuren liegen in dem üblicherweise verwendeten Probenmaterial jedoch in sehr geringen Konzentrationen vor. Aus diesem Grund wurden verschiedene Verfahren zur Isolierung der Nukleinsäuren von anderen Probenbestandteilen, wie Proteinen oder anderen zellulären Bestandteilen, die teilweise die anschließenden Nachweisverfahren stören, erarbeitet. Ein Teil dieser Verfahren verwendet festphasengebundene Fangsonden, die mit den abzutrennenden Nukleinsäuren hybridisieren können und diese an der Festphase zurückhalten, während die übrigen Probenbestandteile entfernt werden. Ein solches Verfahren ist beispielsweise in EP-B-0 305 399 beschrieben. Diese Verfahren haben jedoch den Nachteil, daß sie sich jeweils nur für die Reinigung von Nukleinsäuren mit einer ganz speziellen Nukleotidsequenz eignen.

In WO 91/12079 ist ein Verfahren für die Isolierung von Nukleinsäuren mit Hilfe von Magnetpartikeln aus Zellulose und Eisenoxid beschrieben, wobei die Partikelgröße mit zwischen 1 und 10 µm angegeben ist. Diese Partikel enthalten keine Glasoberfläche und sind nur für eine Isolierung unter Präzipitation von Nukleinsäuren geeignet. Durch Aggregation werden jedoch eine Vielzahl von Probenbestandteilen eingeschlossen, die spätere Verfahrensschritte stören.

In EP-B-0389 063 wird ein Verfahren vorgeschlagen, bei dem die Probe mit einem Gemisch eines chaotropen Guanidiniumsalzes und Silicapartikeln gemischt wird. Unter diesen Bedingungen binden Nukleinsäuren relativ sequenzunabhängig an die Silica-Oberfläche. Die übrigen Probenbestandteile können abgewaschen und die Nukleinsäuren anschließend eluiert werden.

In WO 96/41811 werden magnetische Partikel mit einer im wesentlichen porenfreien Glasoberfläche zur sequenzunabhängigen Reinigung von Nukleinsäuren beschrieben. Die dort verwendeten Partikel haben einen Kern, bevorzugt mit Magnetit als magnetischem Werkstoff und haben eine bevorzugte Korngröße von zwischen 10 und 60 µm. Magnetit in Kristallen größer ca. 30 bis 50 nm zeigt hartmagnetische Eigenschaften. Durch ein externes Magnetfeld wird permanenter Magnetismus induziert. Partikel mit solchen hartmagnetischen Kernen zeigen nach dem ersten Einwirken eines externen magnetischen Feldes die Eigenschaften eines kleinen Permanentmagneten. In Suspension ziehen sich solche Partikel gegenseitig an und bilden größere Einheiten. Diese größeren Einheiten sedimentieren unter dem Einfluß eines äußeren Schwerefeldes schneller, als die Partikel als Einzelne. Das ist nachteilig, da länger andauernde Inkubationen häufiges Redispergieren erfordern.

In WO 96/41840 werden Pigmente beschrieben, die eine Glasoberfläche einer Dicke von mindestens 0,8 µm aufweisen. Als eine glasbildende Komponente werden auch Verbindungen von Zink vorgeschlagen. Es entstehen dabei Pigmentpartikel mit einer Teilchengröße von vorzugsweise 2 bis 20 µm.

Es hat sich nun herausgestellt, daß bei den bisher beschriebenen Verfahren zur Herstellung von Partikeln nach dem Sol-Gel-Prozeß, bei dem Kernpartikel einer vorgegebenen Größe mit einem Gel beschichtet werden und anschließend eine Verdichtung zu einer Glasoberfläche stattfindet, ein hoher Anteil von Partikeln gebildet wird, die kein Kernpartikel enthalten. Dies führt entweder dazu, daß bei mit solchen Zubereitungen durchgeführten Nukleinsäurenachweisverfahren große Verluste an Nukleinsäuren stattfinden oder die Feinanteile zur Erhöhung der Ausbeute aufwendig abgetrennt werden müssen. Aufgabe der vorliegenden Erfindung war es, den vorliegenden Stand der Technik ganz oder teilweise zu verbessern, insbesondere Partikel mit einer relativ engen Korngrößenverteilung herzustellen und die Ausbeute bei Nukleinsäurereinigung weiter zu erhöhen, oder/und Partikel für die Nukleinsäurereinigung bereitzustellen, die auch nach der Einwirkung eines äußeren Magnetfeldes nur sehr geringe Tendenz zur Zusammenlagerung zeigen und genauso langsam im Schwerefeld sedimentieren, wie solche, die noch nie einem Magnetfeld ausgesetzt waren.

Gegenstand der Erfindung ist eine Zubereitung enthaltend Partikel mit einer Glasoberfläche, wobei mehr als 75 Gew.% dieser Partikel eine Korngröße von zwischen 0.5 und 15 µm haben.

Weitere Gegenstände der Erfindung sind ein Verfahren zur Herstellung einer Zubereitung von Partikeln enthaltend einen Kern ummantelt mit einer Gelschicht oder einer Glasschicht und ein Verfahren zur Reinigung von Nukleinsäuren mit Hilfe der erfindungsgemäßen Zubereitung.

Ein weiterer Gegenstand der Erfindung ist ein Verfahren zur Herstellung und eine Zubereitung von Partikeln mit einem superparamagnetischen Kern.

Ein weiterer Gegenstand der Erfindung ist ein Verfahren zur Herstellung und eine Zubereitung von Partikeln mit einem magnetischen, bevorzugt einem weichmagnetischen metallischem Kern.

Als Partikel bezeichnet der Fachmann feste Materialien mit einem geringen Durchmesser. Bevorzugt haben diese Partikel eine im wesentlichen kugelige Oberfläche. Hier sollen aber auch plättchenförmige und fadenförmige Teilchen in den unten angegebenen Dimensionen als Partikel verstanden werden. Um für die Reinigung von Nukleinsäuren besonders gut geeignet zu sein, ist es wünschenswert, daß das Partikel einen Kern (Pigmentanteil) aufweist, der bevorzugt magnetisch ist und der mit einer Glasschicht ummantelt ist. Solche Kerne enthalten bevorzugt Metalloxide, wie Aluminiumoxid, Eisenoxid, Chromoxid, Kupferoxid, Manganoxid, Bleioxid, Zinnoxid, Titanoxid, Zinkoxid und Zirkoniumoxid oder Metalle, wie Fe, Cr, Ni oder magnetische Legierungen. Die Zusammensetzung dieses Kerns ist für die Funktion der erfindungsgemäßen Partikel weniger wesentlich, da der Kern mit einer Glasoberfläche ummantelt wird, so daß der Kern nicht direkt mit der Probe, aus der die Nukleinsäure isoliert werden soll, in Berührung kommt. Solche Kerne sind kommerziell erhältlich. Sofern der Kern Fe₃O₄ (Magnetit) oder Fe₂O₃ (Maghämit) oder Fe oder Cr oder Ni oder magnetische Legierungen enthält, sind diese Kerne magnetisch.

Als Materialien, die als weichmagnetisch bezeichnet werden, kommen Metalle auf Basis der reinen Elemente Fe, Ni, Cr und Legierungen davon, bevorzugt auf der Basis von Ni, in Frage. Beispiele solcher Legierungen sind unter der Bezeichnung Permalloy bekannt. Sie bestehen zu 70 bis 80 % aus Ni mit Zusätzen von Cr, Cu und Mo. Partikel aus magnetisch weichem Material ziehen einander in Abwesenheit eines externen Magnetfeldes nicht oder nur vernachlässigbar an.

Feinteilige Metallpulver sind sehr reaktiv. An Luft besteht die Gefahr der Selbstentzündung, sie gelten als pyrophor. Daher war es sehr überraschend, daß solch feinteilige Metallpartikel nach dem Sol-Gelverfahren mit einer Glasschicht überzogen werden konnten, ohne daß sich die magnetischen Eigenschaften wesentlich änderten. Besonders bevorzugt werden als Metallpulver Carbonyleisenpulver eingesetzt, wobei in H₂ reduzierte Typen magnetisch besonders bevorzugte Eigenschaften aufweisen. Bevorzugte Eigenschaften weisen auch Caronyleisenwhisker auf.

Metallpulver haben bevorzugt eine Korngröße von zwischen 10 nm und 100 µm, besonders bevorzugt von zwischen 200 nm und 8 µm.

Eine Glasoberfläche im Sinne der vorliegenden Erfindung besteht aus einem siliziumhaltigen amorphen Material. Das Glas enthält bevorzugt neben Siliziumoxid einen oder mehrere der folgenden Komponenten (in mol%):
B₂O₃ (0-30%), Al₂O₃ (0-20 %), CaO (0-20 %), BaO (0-10 %), K₂O (0-20 %), Na₂O (0-20 %), MgO (0-18 %), Pb₂O₃(0-15 %), ZnO (0-6 %).

In geringerem Umfang von 0-5 % können auch eine Vielzahl anderer Oxide, wie z. B. Na₂O, Mn₂O₃, TiO₂, As₂O₃, Fe₂O₃, CuO, ZrO₂, CoO usw. enthalten sein. Als besonders wirksam haben sich Oberflächen einer Zusammensetzung von SiO₂, B₂O₃, Al₂O₃, CaO, K₂O, und ZnO erwiesen. Unter dem Gesichtspunkt der Ausbeute an Nukleinsäuren besonders bevorzugte Borsilikatgläser haben einen Zinkoxidgehalt von 2-6, bevorzugt von ca. 4 mol%. Besonders bevorzugt besteht die Glasschicht aus 68-79 mol% SoO₂, 15-5 mol% B₂O₃, 6-2.5 mol% Gesamtmenge an K₂O und Na₂O, 4-1 mol% CaO, 8-2 mol% Al₂O₃, 6-2 mol% ZnO. Besonders bevorzugt im Sinne der Erfindung sind Gläser, die durch den sogenannten Gel-Sol-Prozeß und anschließendes Trocknen und Verdichten der gebildeten Schicht gebildet werden. Dieser Prozeß ist in seinen Grundzügen bekannt und wurde z. B. in C.J. Brinker, G.W. Scherer "Sol Gel science - The physics and chemistry of Sol Gel Processing", Academic Press Inc. 1990 und Sol-Gel Optics, Processing and Applications Lisa C. Klein Ed. Kluwer Academic Publishers 1994, Seite 450 ff. sowie in DE-A-1941191, DE-A-3719339, DE-A-4117041 und DE-A-4217432 beschrieben. Im Gel-Sol-Prozeß werden Alkoxide von netzwerksbildenden Komponenten, z. B. SiO₂, B₂O₃, Al₂O₃, TiO₂, ZrO₂ und ZnO zusammen mit Oxiden und Salzen anderer Komponenten, z. B. in alkoholischer Lösung, vorgelegt und hydrolysiert.

Durch die Zugabe von Wasser wird der Hydrolyseprozeß der Ausgangskomponenten in Gang gesetzt. Die Reaktion verläuft relativ rasch, da die Alkaliionen katalytisch auf die Hydrolysegeschwindigkeit des Kieselsäureesters einwirken. Nach Ablauf der Gelbildung kann das entstehende Gel getrocknet und durch einen thermischen Prozeß zu einem Glas verdichtet werden.

Das Mengenverhältnis Sol/Pigment hat einen erheblichen Einfluß auf die Ausbeute an erfindungsgemäßen magnetischem Pigment. Grenzen sind dadurch gegeben, daß der Pigmentanteil so gering ist, daß eine noch pump- und sprühfähige Masse entsteht. Bei zu geringem Pigmentanteil wird der Feinanteil, z. B. von nicht-magnetischem Material zu groß und stört. Als im Hinblick auf die Pigmentausbeute zweckmäßige Mengenverhältnisse wurden 10 bis 45 g Pigment/100 ml Sol gefunden.

Die Aufschlämmung wird zur Entstehung eines Pulvers bevorzugt durch eine Düse versprüht und das Aerosol auf einer Fallstrecke getrocknet Die Düse wird bevorzugt geheizt, um die Trocknung der Aufschlämmung zu beschleunigen. Abhängig von der Geometrie der Düse beträgt die Düsentemperatur bevorzugt ca. 120 bis 250 °C. Ein Kompromiß wird gefunden durch ausreichende Verdampfungsgeschwindigkeit, jedoch Vermeiden von Verspritzen.

Im Hinblick auf die Ausbeute ist die Verdichtungstemperatur möglichst hoch zu wählen. Ist sie jedoch zu hoch, verkleben die Partikel untereinander und es bilden sich Agglomerate, die herausgesiebt werden sollten. Ein Zusatz von Zink in der Schicht erhöht jedoch überraschenderweise den Schmelzpunkt, so daß eine höhere Verdichtungstemperatur (zwischen 710 und 800 °C) möglich ist. Die Nachbehandlung unter Luft führt bei zu hohen Temperaturen zu einem Verlust der magnetischen Eigenschaften, weshalb zu hohe Temperaturen vermieden werden sollten. Auch hier sind bei Zusatz von Zink andere Temperaturen möglich (bevorzugt zwischen 150 und 250 °C).

Es hat sich im Rahmen der vorliegenden Erfindung gezeigt, daß in den in WO 96/41811 beschriebenen Verfahren als magnetische Kerne auch solche eingesetzt werden können, die wesentlich kleiner sind. Insbesondere zeigte es sich, daß es möglich ist, nanoscalige Kerne, z.B. Magnetit, mit einer Kristallgröße kleiner 50 nm, bevorzugt kleiner 30 nm, einzusetzen. Die untere Grenze der Kerngröße ergibt sich aus der Handhabbarkeit der Kerne, besonders ihrer Neigung zur Bildung von Aggregaten. Bevorzugt sind die Kerne größer als 5 nm, besonders bevorzugt als 7 nm. Das magnetische Verhalten der nanoscaligen Kerne wird als superparamagnetisch bezeichnet. Die erhaltenen Partikel sedimentieren rasch unter Einwirkung eines externen Magnetfeldes. Nach Redispersion unterscheidet sich die Sedimentationsgeschwindigkeit im Schwerefeld nicht von der Sedimentationsgeschwindigkeit im Schwerefeld vor der Einwirkung des externen Magnetfeldes. Vorteilhaft dabei ist, daß längere Inkubationszeiten in Suspension möglich sind, ohne daß wieder aufgemischt und resuspendiert werden muß.

Zur Herstellung der erfindungsgemäßen Zubereitung wird eine Zubereitung von Kernpartikeln, in der mehr als 75 Gew.% der Kempartikel eine Korngröße von zwischen etwas weniger als 0.5 und etwas weniger als 15 µm haben, in den Sol/Gel-Prozeß eingesetzt. Die Kernpartikel müssen um soviel kleiner sein als die glasummantelten Partikel, wie die Dicke der Glasschicht ausmacht. Die Glasschicht wird nach dem erfindungsgemäßen Verfahren zwischen 5 nm und 1 µm dick sein, abhängig von den jeweils gewählten Umständen, wie Verhältnis Gel zu Kernpartikel. Im Durchschnitt dürfte die Glasschicht zwischen 0.2 und 0.3 µm dick sein.

Besonders bevorzugt ist eine Zubereitung enthaltend Partikel mit einer Glasoberfläche wobei mehr als 75 Gew.% dieser Partikel eine Korngröße von zwischen 2 und 15 µm haben. Besonders bevorzugt ist der Anteil der Partikel mit der bestimmten Korngröße größer als 90 Gew.%.

Besonders bevorzugt werden magnetische Kernpartikel eingesetzt. Die erfindungsgemäße Zubereitung hat den Vorteil, daß bevorzugt mehr als 95 Gew.% der Partikel mit einer Korngröße von zwischen 0.5 und 15 µm, bevorzugt zwischen 2 und 15 µm magnetisch sind. Dies bedeutet, daß gegenüber den bekannten Verfahren der Anteil von nicht-kernhaltigen Partikeln drastisch reduziert ist. Dies kann man daran erkennen, nur wenig nicht-magnetische Partikel enthalten sind. Dies führt dazu, daß es praktisch nicht mehr erforderlich ist, die gebildeten nicht-magnetischen Partikel von den magnetischen Partikeln abzutrennen, bevor die Zubereitung in Verfahren zur Reinigung von Nukleinsäuren eingesetzt wird. Dies bedeutet eine Vereinfachung im Herstellprozeß.

Außerdem kann die erfindungsgemäße Zubereitung dadurch charakterisiert werden, daß bevorzugt weniger als 50 % der Partikel eine Korngröße von weniger als 2 µm haben. Dies hat zur Folge, daß der nicht-magnetische Feinanteil, der bei kleinen Korngrößen einen hohen relativen Anteil ausmacht, stark reduziert ist. Besonders bevorzugt haben weniger als 2 % der Partikel eine Korngröße von weniger als 0.5 µm.

Bevorzugt sind nicht mehr als 10 %, besonders bevorzugt zwischen 10 und 40 % der Partikel der Zubereitung Partikel mit einer Korngröße von mehr als 10 µm.

Die erfindungsgemäße Zubereitung kann neben den erfindungsgemäßen Partikeln noch weitere nicht-glashaltige Bestandteile enthalten, wie z. B. Puffersubstanzen oder ein Suspensionsmittel, z. B. Wasser oder alkoholische Lösungen von Wasser.

Die Glasschicht der Partikel der erfindungsgemäßen Zubereitung enthält bevorzugt einen Anteil von zwischen 2 und 6 mol%, besonders bevorzugt 4 mol% Zinkoxid. Dies kann dadurch erreicht werden, daß der Anteil von Zinkoxid an der festen Masse des Sols, verglichen mit den Anteilen der übrigen festen Komponenten, in diesem Größenordnungsbereich liegt. Der Anteil von Zinkoxid vergrößert sich mit Reduzierung des Anteils von Boroxid, insbesondere bei längerem Erhitzen, da Boroxid unter den Herstellbedingungen schon flüchtig ist.

Partikel, die eine Glasschicht aufweisen, in der der Anteil von Zinkoxid zwischen 2 und 6 mol% liegt, haben sich als besonders wirksam bei der Reinigung von Nukleinsäuren herausgestellt. Die Ausbeute an Nukleinsäuren konnte, verglichen mit derselben Glasschicht ohne Zinkoxid, teilweise um 50 % erhöht werden.

Ebenfalls Gegenstand der Erfindung ist ein Verfahren zur Herstellung einer Zubereitung von Partikeln mit einem Kern ummantelt mit einer Gelschicht enthaltend weniger als 5 Gew.% kernlose Partikel enthaltend die Schritte Suspendieren von Kernpartikeln in einem Sol unter Verwendung einer Kernpartikelzubereitung und Sprühtrocknen der Suspension unter Gelbildung, wobei die Kernpartikelzubereitung zu 75 Gew.% aus Partikeln mit einer Korngröße von zwischen 0.5 und 15 µm, bevorzugt zwischen 2 und 15 µm besteht.

Zur Durchführung des Gel/Sol-Prozesses, welchen das erfindungsgemäße Herstellungsverfahren benutzt, wird auf die Beschreibung im Stand der Technik verwiesen. Der wesentliche Unterschied der Erfindung zu dem Vorbeschriebenen, ist der Einsatz einer bestimmten Kernpartikelzubereitung, durch welche eine Zubereitung mit weniger als 5 Gew.% kernloser Partikel hergestellt werden kann. Als besonders vorteilhaft hat sich ein Verfahren erwiesen, bei dem zunächst ein Sol aus Tetraalkylorthosilikaten, Alkylboraten, Aluminiumalkoholaten und Alkalialkoholaten in Ethanol hergestellt wird und diese Mischung mit Kalzium erhitzt wird. Anschließend wird die Mischung durch Zusatz von Wasser hydrolysiert. In das so gebildete Sol werden die Kernpartikel in Festform zugegeben und, bevorzugt mit Ultraschall, suspendiert. Anschließend wird die Suspension unter Gelbildung in einem Sprüh-Trocknungsverfahren, bei dem die Düse geheizt wird, und bei dem im wesentlichen Partikel entstehen, in denen 1 bis nur wenige Kempartikel pro Partikel enthalten sind (bevorzugt enthalten weniger als 1 % der Partikel mehr als 10 Kernpartikel), versprüht. Das Sprühprodukt wird anschließend erhitzt, um das Gel zu einem Glas zu verdichten. Auch hier hat der Zusatz von Zinkoxid zum Gel einen erheblichen Vorteil. Die Verdichtung kann bei höheren Temperaturen als bei solchen ohne Zinkzusatz durchgeführt werden, da der Erweichungspunkt des entstehenden Glases höher liegt.

Dadurch lassen sich organische Restbestandteile aus den eingesetzten Materialien leichter austreiben.

Da nach dem erfindungsgemäßen Verfahren eine Zubereitung mit einem sehr geringen Anteil von kernlosen Partikeln entsteht, ist im allgemeinen keine anschließende Fraktionierung nach kernlosen/kernhaltigen Partikeln mehr erforderlich.

Ebenfalls Gegenstand der Erfindung ist ein Verfahren zur Reinigung von Nukleinsäuren durch nicht-kovalente Bindung der Nukleinsäuren aus einer Probe an Partikel mit einer Glasoberfläche, Entfernen nicht-gebundener Probenbestandteile und Elution der gebundenen Nukleinsäuren von der Glasoberfläche, wobei eine erfindungsgemäße Zubereitung eingesetzt wird. Das Verfahren wird besonders einfach, wenn die Partikel magnetisch sind.

Verfahren zur Reinigung von Nukleinsäuren mit Hilfe magnetischer Partikel mit einer Glasoberfläche sind in WO 96/41811 beschrieben. Auf diese Offenbarung wird hier voll inhaltlich Bezug genommen. Als Proben für das erfindungsgemäße Reinigungsverfahren kommen insbesondere klinische Proben, wie Blut, Serum, Mundspülflüssigkeit, Urin, Zerebralflüssigkeit, Sputum, Stuhl, Plasma, Punktate oder Knochenmarksproben in Frage. Bevorzugtes Probenmaterial ist Serum. Zur Reinigung der Nukleinsäuren wird die Probe, erforderlichenfalls nach Lyse eventuell enthaltender zellulärer Strukturen und Verdau von strörenden Probenbestandteilen, mit der erfindungsgemäßen Zubereitung versetzt, z. B. in Form einer bestimmten Menge einer Suspension der Partikel. Nach einer Inkubationszeit, während derer die Nukleinsäuren an die Glasoberfläche sequenzunspezifisch binden, wird die Flüssigkeit zusammen mit den nicht-gebundenen Probenbestandteilen entfernt und die Partikel gewünschtenfalls gewaschen, um Reste zu entfernen. Die noch daran gebundenen Nukleinsäuren werden durch Elution mit einer Flüssigkeit, in der sich die Nukleinsäuren gut lösen, von der Oberfläche entfernt. Die resultierende Flüssigkeit kann nun beliebig weiter bearbeitet werden, insbesondere in Amplifikationsverfahren eingesetzt werden, wie z. B. die PCR, da während des Reinigungsverfahrens die meisten Enzyminhibitoren abgetrennt wurden.

Sofern die Partikel magnetisch sind, ist die Entfernung der Flüssigkeit von Partikeln mit den Nukleinsäuren besonders einfach, da die Partikel mit Hilfe eines Magneten gesammelt und festgehalten werden können, während die Flüssigkeit entfernt wird. Wenn die Partikel nicht magnetisch sind, können sie von der Flüssigkeit durch Filtration mit einem geeigneten Filter abgetrennt werden.

Die vorliegende Erfindung wird durch die nachfolgenden Beispiele näher erläutert.

### Beispiel 1

### Sol zur Herstellung einer zinkfreien Schicht (74 SiO₂ x 15B₂O₃ x 4K₂O x 2CaO x 5Al₂O₃)

In einen 5 Liter Rundkolben werden 1750 ml Tetraethylorthosilikat (Hersteller: Wacker, Burghausen) vorgelegt und bei Raumtemperatur unter Rühren (500 U/min) zügig zugegeben:
541 ml Triethylborat (Hersteller: Aldrich, Steinheim)
250 ml Kaliummethanolat (25%ig in Methanol (Hersteller: Fluka, Deisenhofen))
261 g Aluminium-sec.-Butylat (Hersteller: Aldrich, Steinheim)
292 ml Ethanol und
8,49 g Calcium (Hersteller: Fluka, Deisenhofen)

Die Mischung wird anschließend unter Rühren erhitzt bis zum starken Rückfluß. Über 30 Minuten wird eine Mischung von insgesamt 583 ml Ethanol und 233 ml Wasser zugetropft. Nach Abkühlen auf < 50 °C wird das Sol umgefüllt in einen offenen Behälter und 1200 g Pigment IRIODIN 600 Black Mica (Hersteller: Merck, Darmstadt) dazugegeben. Das Sol wird nach vollständiger Pigmentzugabe noch 1 Minute bei 500 U/min gerührt und anschließend 5 Minuten mit Ultraschall behandelt. Nach der Ultraschallbehandlung wird das Sol-Pigment-Gemisch mit einem Dissolverrührer bei ca. 500 U/min gerührt, bis die gesamte Menge aufgebraucht ist.

### Beispiel 2

### Herstellung von glasbeschichtetem Pigment (MGP)

Versprüht wird in einem Sprühturm der Fa. Nubilosa, Konstanz, mit einem Durchmesser von 0,75 m, einer Höhe von 2,5 m und einer Verdunstungsleistung (bezogen auf Wasser) von 1 - 3 Liter/Stunde. Die Lufteinlaßtemperatur beträgt 270 °C, die Auslaßtemperatur ca. 130 °C. Der Durchsatz der Luft ist 7,2 m³/min. Zum Sprühen wird eine Zweistoffdüse verwendet mit einem Sprühdruck von 2 bar. Die Förderleistung der verwendeten Kugelventilmembranpumpe beträgt 60 g Sol/min.

Das Sprühprodukt wird in einem Zyklon abgefangen, an Luft bei 250 °C 1 Stunde vorverdichtet und anschließend in einem Stickstoffofen mit einer Heizrate von 1 K/min auf eine Temperatur von 675 °C gebracht, 1 Stunde dort gehalten und abgekühlt auf 300 °C. Bei 300 °C wird Sauerstoff zudosiert, 1 Stunde gehalten, dann abgekühlt auf Raumtemperatur. Nach dem Abkühlen erfolgt eine Siebung mit einem Sieb mit einer Maschenweite von 50 µm zur Entfernung von eventuell vorhandenen Aggregaten. Damit ist die Herstellung abgeschlossen.

### Beispiel 3

### Sol zur Herstellung einer zinkhaltigen Schicht (70,67 SiO₂ x 14,33 B₂O₃ x 4 K₂O x 2 CaO x 5 Al₂O₃ x 4 ZnO)

In gleicher Weise wie in Beispiel 1 wird ein zinkhaltiges Sol bereitet. Dazu werden folgende Edukte eingewogen und analog behandelt:
1258 ml Tetraethylorthosilikat (Hersteller: Wacker, Burghausen)
387 ml Triethylborat (Hersteller. Aldrich, Steinheim)
188 ml Kaliummethanolat 25%ig in Methanol (Hersteller: Fluka, Deisenhofen)
196 g Aluminium-sec.-Butylat (Hersteller: Aldrich, Steinheim)
1285 ml Ethanol
6,39 g Calcium (Hersteller: Fluka. Deisenhofen)
58,5 g Zinkacetat dehydriertes Dihydrat (Hersteller: Fluka, Deisenhofen)

Nach dem Kochen unter Rückfluß werden 178 ml H₂O zusammen mit 444 ml Ethanol binnen 30 Minuten zugetropft. Nach dem Abkühlen werden 1200 g Pigment zugesetzt. Ansonsten siehe Beispiel 1.

### Beispiel 4

### Herstellung von zinkhaltigen glasbeschichtetem Pigment

Das pigmenthaltige Sol aus Beispiel 3 wird analog Beispiel 2 verarbeitet. Die Verdichtungstemperatur beträgt jedoch 750 °C.

### Beispiel 5

### Herstellung von zinkhaltigen glasbeschichtetem Pigment mit modifizierter Nachbehandlung (MGP, Magnetische Glas-Partikel)

Das pigmenthaltige Sol aus Beispiel 3 wird analog Beispiel 2 verarbeitet. Die Verdichtungstemperatur beträgt jedoch 750 °C und die Temperatur bei der Behandlung in Sauerstoff beträgt 200 °C.

### Beispiel 6

### Ermittlung der Ausbeute von DNA bzw. RNA mit radioaktivem ³²P

Zum direkten Nachweis von gebundener bzw. nicht-gebundener DNA bzw. RNA werden ³²P-markierter HIVgag RNA Standard mit 1,4 kb bzw. ³²P-markierter Lambdaamplikons mit 3 kb eingesetzt. Als Probe dient Negativplasma (human) enthaltend jeweils 10⁹ Kopien.

### Durchführung der Probenvorbereitung

In ein 2 ml Eppendorf-Gefäß werden 500 µl Negativplasma mit 10⁹ Kopien ³²P gelabelte Lambdaamplikons gegeben. Dazu werden 480 µl Bindepuffer/Proteinase K-Lösung (5:1) zupipettiert, gevortext und bei 70 °C für 10 Minuten inkubiert. Nach Abkühlen auf Raumtemperatur werden 400 µl isopropanolische MGP-Suspension mit einem Gesamtinhalt von 3 mg MGP zupipettiert. Unmittelbar darauf wird durch Vortexen gemischt. Die Probe wird dann für 15 Minuten auf einem Mischer, z. B. Thermomischer 5436 von Eppendorf, inkubiert.

Die MGP werden durch Überführung der Probe in einen Magnetseparator konzentriert. Nach 1 Minute wird der Überstand vollständig abpipettiert.

0,5 ml Waschpuffer werden zu den MGPs pipettiert. Die Probe wird gevortext und dann in den Magnetseparator überführt. Der Überstand wird nach 1 Minute abpipttiert. Die Waschprozedur wird noch 2 x wiederholt.

Zu den MGP werden 200 ml Elutionspuffer zugesetzt. Bei 80 °C wird 10 Minuten auf einem Thermomischer bei 1400 RPM inkubiert. Die Probe wird in den Magnetseparator überführt und nach 1 Minute wird das gesamte Eluat abgenommen. Das Eluat wird dann in ein neues Gefäß überführt und in einem Szintilationszähler vermessen.

Aus dem Verhältnis der Radioaktivität des Eluates zu der Radioaktivität der Probe vor der Reinigungsprozedur kann die Ausbeute ermittelt werden.

Ergebnisse mit MGPs unterschiedlicher Beschichtung:

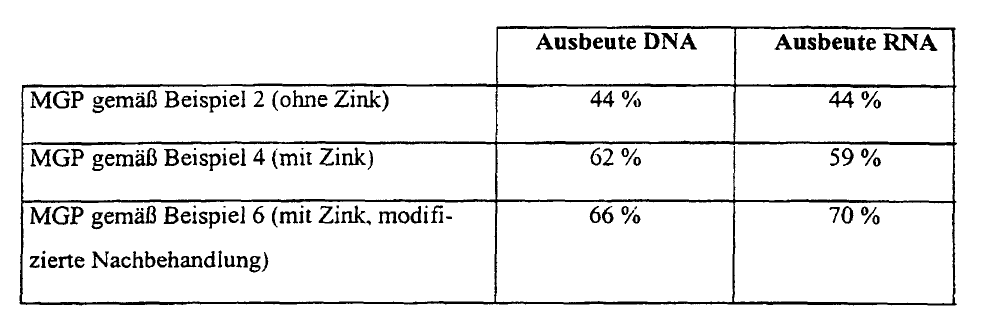

### Beispiel 7

### Black Mica (BM) als Pigmentbasis (Referenzbeispiel)

Gemäß Beispiel 1 wird eine Charge gefertig, bei der das Pigment Black Mica (BM) ist.

### Beispiel 8

### Microna Matte Black (MMB) als Pigmentbasis

Gemäß Beispiel 1 wird eine Charge gefertigt, bei der das Pigment MMB (Microna Matte Black (Hersteller: Merck, Darmstadt)) ist.

### Beispiel 9

### Signalhöhe nach Amplifikation bei Probenvorbereitung mit MGP auf verschiedener Pigmentbasis

MGP gemäß den Beispielen 7 und 8 wird bei einer Probenvorbereitung eingesetzt. Als Probe dient Humanplasma mit 100 Kopien/ml HCV-Viren. Das Eluat der Probenvorbereitung wird einer Amplifikation unterworfen und das Amplifikationsergebnis mit einem Elektrolumineszenzverfahren detektiert. In einem weiteren Versuch war die Probe Humanplasma mit 600 Kopien/ml HBV-Viren.

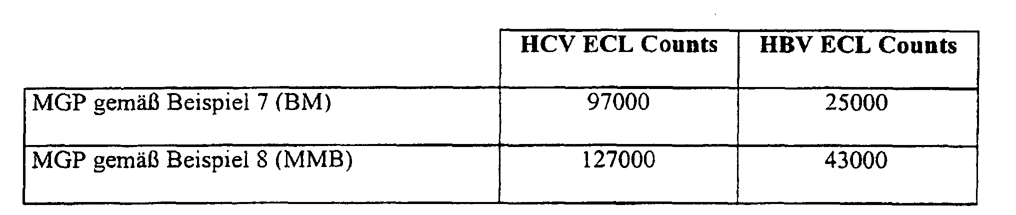

### Beispiel 10

### Carbonyleisenpulver HQ als Pigment

Ein zinkhaltiges Sol wird in Analogie zu Beispiel 3 hergestellt. jedoch nur 240 g Sol.

Nach dem Abkühlen werden 71 g Carbonyleisenpulver HQ (BASF, Ludwigshafen), mit der Korngrößenverteilung: 10% < 0,5 µm, 50% < 1,1 µm, 90% < 2,2 µm, zugesetzt, 1 Minute bei 500 U/min gerührt und anschließend 5 Minuten mit Ultraschall behandelt. Das Sol wird in einem Sprühtrockner (Büche 190, Mini Spray Dryer) versprüht. Die Düsentemperatur des Sprühtrockners beträgt 140 °C.

Das erhaltene Pulver wird bei 150 °C an Luft ausgeheizt. Die Aufheizgeschwindigkeit beträgt 1 K/min, die Haltezeit 1 Stunde. Anschließend wird die Luft im Ofen durch N₂ ersetzt, mehrfach gespült und mit 1 K/min auf 700 °C erhitzt, 1 Stunde gehalten, auf 200 °C abgekühlt mit 1 K/min. Bei 200 °C wird N, durch Luft ersetzt und 1 Stunde gehalten. Danach wird abgekühlt auf Raumtemperatur. Eventuell entstandene Aggregate werden mit einem 50 µm Sieb ausgesiebt.

Die Ausbeute beträgt 62,4 g. Siebverluste sind vernachlässigbar: Aggregate treten nicht auf.

### Beispiel 11

### Ermitteln der Bindung von RNA

Gemäß Beispiel 6 wird ³²P-markierter HIVgag Standard mit 1,4 Kb an die Partikel gemäß Beispiel 10 gebunden. Die radioaktive Messung ergibt eine Bindung > 80 %.

### Beispiel 12

### Vergleich der Sedimentationsgeschwindigkeiten

Je 3 mg Partikel gemäß Beispiel 10 werden in zwei Eppendorf-Gefäße mit einem Volumen von 2 ml überrührt und mit je 1,5 ml H₂O suspendiert.

Die Partikel in Gefäß 1 werden mit einem Magneten an dic Gefäßwand gezogen und anschließend durch Schütteln resuspendiert. Gleichzeitig werden die Partikel in Gefäß 2 aufgeschüttelt.

Die Sedimentation im Schwerefeld wird visuell beobachtet. Es ergeben sich keine Unterschiede.

## Patentansprüche

1. Zubereitung enthaltend Partikel mit einer Glasoberfläche, wobei mehr als 75 Gew.% dieser Partikel eine Korngröße von zwischen 0.5 und 15 µm haben
**dadurch gekennzeichnet, daß**
die Glasoberfläche sich aus SiO₂, B₂O₃, K₂O, CaO, Al₂O₃ und ZnO zusammensetzt.

2. Zubereitung gemäß Anspruch 1 **dadurch gekennzeichnet, daß** mehr als 95 Gew.% der Partikel mit einer Korngröße von zwischen 0.5 und 15 µm magnetisch sind.

3. Zubereitung gemäß Anspruch 1 oder 2, **dadurch gekennzeichnet, daß** weniger als die Hälfte der Partikel eine Korngröße von weniger als 2 µm haben.

4. Zubereitung nach einem der Ansprüche 1,2 und 3, **dadurch gekennzeichnet, daß** weniger als 2 % der Partikel eine Korngröße von weniger als 0.5 µm haben.

5. Zubereitung gemäß Anspruch 2, **dadurch gekennzeichnet, daß** die magnetischen Partikel einen magnetischen Kern aufweisen, der mit Glas ummantelt ist.

6. Zubereitung gemäß Anspruch 1, **dadurch gekennzeichnet, daß** nicht mehr als 10 % dieser Partikel Partikel mit einer Korngröße von mehr als 10 µm sind.

7. Zubereitung gemäß einem der vorangehenden Ansprüche, **dadurch gekennzeichnet, daß** die Partikel einen Glasmantel aufweisen, der einen Anteil von zwischen 2 und 6 mol% Zinkoxid enthält.

8. Zubereitung gemäß einem der vorangehenden Ansprüche, enthaltend mindestens einen Kern aus einem magnetischen Metall.

9. Zubereitung gemäß Anspruch 8, **dadurch gekennzeichnet, daß** der oder die Kerne eine Korngröße von zwischen 0,01 µm und 100 µm, besonders bevorzugt zwischen 0,2 µm und 8 µm haben

10. Verfahren zur Herstellung einer Zubereitung von Partikeln mit einem Kern ummantelt mit einer Gelschicht enthaltend weniger als 5 Gew.% kernlose Partikel und wobei die Gelschicht sich aus SiO₂, B₂O₃, K₂O, CaO, Al₂O₃ und ZnO zusammensetzt, enthaltend die Schritte
- Suspendieren von Kernpartikeln in einem Sol unter Verwendung einer Kernpartikelzubereitung
- Sprühtrocknen der Suspension unter Gelbildung
**dadurch gekennzeichnet, daß** die Kernpartikelzubereitung zu 75 Gew.% aus Partikeln mit einer Korngröße von zwischen 0.5 und 15 µm besteht.

11. Verfahren zur Reinigung von Nukleinsäuren durch nicht-kovalente Bindung der Nukleinsäuren aus einer Probe an Partikel mit einer Glasoberfläche enthaltend ZnO, Entfernen nicht-gebundener Probenbestandteile und Elution der gebundenen Nukleinsäuren von der Glasoberfläche.

12. Verfahren gemäß Anspruch 11, **dadurch gekennzeichnet, daß** die Probe mit einer Zubereitung gemäß einem der Ansprüche 1 bis 9 in Kontakt gebracht wird.

13. Verfahren gemäß einem der Ansprüche 11 oder 12, **dadurch gekennzeichnet, daß** die Partikel magnetisch sind und bei Entfernung der Probenbestandteile durch einen Magneten festgehalten werden.

14. Verfahren zur Herstellung einer Zubereitung von Partikeln mit einem Kern ummantelt mit einer Glasschicht enthaltend weniger als 5 Gew.% kernlose Partikel und wobei die Glasoberfläche sich aus SiO₂, B₂O₃, K₂O, CaO, Al₂O₃ und ZnO zusammensetzt, enthaltend die Schritte
- Suspendieren von Kernpartikeln in einem Sol unter Verwendung einer Kernpartikelzubereitung
- Sprühtrocknen der Suspension unter Gelbildung
- Verdichtung des Gels zum Glas
**dadurch gekennzeichnet, daß** die Kernpartikelzubereitung zu 75 Gew.% aus Partikeln mit einer Korngröße von zwischen 0.5 und 15 µm besteht

15. Verwendung von Zinkoxid in nach dem Sol/Gel-Prozeß aus Anspruch 14 erzeugten Glasschichten zur Erhöhung der Bindefähigkeit der Glasoberfläche für Nukleinsäuren.

## Claims

1. Preparation containing particles having a glass surface, more than 75 % by weight of said particles having a particle size between 0.5 and 15 µm, wherein the glass surface is composed of SiO₂, B₂O₃, K₂O, CaO, Al₂O₃ and ZnO.

2. Preparation as claimed in claim 1, wherein more than 95 % by weight of the particles having a particle size between 0.5 and 15 µm are magnetic.

3. Preparation as claimed in claim 1 or 2, wherein less than half of the particles have a particle size of less than 2 µm.

4. Preparation as claimed in one of the claims 1,2 and 3, wherein less than 2 % of the particles have a particle size of less than 0.5 µm.

5. Preparation as claimed in claim 2, wherein the magnetic particles have a magnetic core which is coated with glass.

6. Preparation as claimed in claim 1, wherein no more than 10 % of these particles are particles with a particle size of more than 10 µm.

7. Preparation as claimed in one of the previous claims, wherein the particles have a glass coat which contains between 2 and 6 mole % zinc oxide.

8. Preparation as claimed in one of the previous claims, containing at least one core of a magnetic metal.

9. Preparation as claimed in claim 8, wherein the core or cores have a particle size of between 0.01 µm and 100 µm, particularly preferably between 0.2 µm and 8 µm.

10. Process for producing a preparation of particles having a core coated with a gel layer containing less than 5 % by weight particles without a core and said gel layer being composed of SiO₂, B₂O_{3,} K₂O, CaO, Al₂O₃ and ZnO comprising the steps
- suspending core particles in a sol using a core particle preparation
- spray drying the suspension to form a gel,
wherein the core particle preparation comprises 75 % by weight particles having a particle size between 0.5 and 15 µm.

11. Process for purifying nucleic acids by non-covalently binding nucleic acids from a sample to particles having a glass surface containing ZnO, removing non-bound sample components and eluting the bound nucleic acids from the glass surface.

12. Process as claimed in claim 11, wherein the sample is contacted with a preparation as claimed in one of the claims 1 to 9.

13. Process as claimed in one of the claims 11 or 12, wherein the particles are magnetic and are held by a magnet while the sample components are removed.

14. Process for producing a preparation of particles having a core coated with a glass layer containing less than 5 % by weight particles without a core and said glass surface being composed of SiO₂, B₂O₃, K₂O, CaO, Al₂O₃ and ZnO, comprising the steps
- suspending core particles in a sol using a core particle preparation
- spray drying the suspension to form a gel,
- compressing the gel to form a glass,
wherein the core particle preparation comprises 75 % by weight particles having a particle size between 0.5 and 15 µm.

15. Use of zinc oxide in glass layers generated by a sol/gel process as claimed in claim 14 to increase the binding capacity of the glass surface for nucleic acids.

## Revendications

1. Préparation contenant des particules possédant une surface vitreuse, plus de 75 % en poids de ces particules possédant une granulométrie entre 0,5 et 15 µm, **caractérisée en ce que** la surface vitreuse se compose de SiO₂, B₂O₃, K₂O, CaO, Al₂O₃ et ZnO.

2. Préparation selon la revendication 1, **caractérisée en ce que** plus de 95 % en poids des particules possédant une granulométrie entre 0,5 et 15 µm sont magnétiques.

3. Préparation selon la revendication 1 ou 2, **caractérisée en ce que** moins de la moitié des particules possèdent une granulométrie inférieure à 2 µm.

4. Préparation selon l'une des revendications 1, 2 et 3, **caractérisée en ce que** moins de 2 % des particules possèdent une granulométrie inférieure à 0,5 µm.

5. Préparation selon la revendication 2, **caractérisée en ce que** les particules magnétiques présentent un noyau magnétique qui est enveloppé de verre.

6. Préparation selon la revendication 1, **caractérisée en ce que** pas plus de 10 % de ces particules sont des particules possédant une granulométrie supérieure à 10 µm.

7. Préparation selon l'une quelconque des revendications précédentes, **caractérisée en ce que** les particules présentent une enveloppe en verre qui contient une fraction d'oxyde de zinc entre 2 et 6 moles %.

8. Préparation selon l'une quelconque des revendications précédentes, contenant au moins un noyau constitué d'un métal magnétique.

9. Préparation selon la revendication 8, **caractérisé en ce que** le ou les noyaux possèdent une granulométrie entre 0,01 µm et 100 µm, de manière particulièrement préférée entre 0,2 µm et 8 µm.

10. Procédé pour la préparation d'une formulation de particules comprenant un noyau enveloppé d'une couche de gel contenant des particules exemptes de noyau à concurrence de moins de 5 % en poids et la couche de gel se composant de SiO₂, B₂O₃, K₂O, CaO, Al₂O₃ et ZnO, contenant les étapes consistant à :
- mettre les particules à noyaux en suspension dans un sol en utilisant une préparation de particules à noyaux,
- lyophiliser la suspension pour obtenir un gel,
**caractérisé en ce que** la préparation de particules à noyaux est constituée, à concurrence de 75 % en poids, par des particules dont la granulométrie se situe entre 0,5 et 15 µm.

11. Procédé pour la purification d'acides nucléiques par liaison non covalente des acides nucléiques provenant d'un échantillon à des particules possédant une surface vitreuse contenant de l'oxyde de zinc, par élimination des constituants non liés de l'échantillon et par élution des acides nucléiques liés par rapport à la surface vitreuse.

12. Procédé selon la revendication 11, **caractérisé en ce qu'**on met l'échantillon en contact avec une préparation selon l'une quelconque des revendications 1 à 9.

13. Procédé selon l'une quelconque des revendications 11 ou 12, **caractérisé en ce que** les particules sont magnétiques et sont retenues par un aimant lors de l'élimination des constituants de l'échantillon.

14. Procédé pour la préparation d'une formulation de particules comprenant un noyau enveloppé d'une couche vitreuse contenant des particules exemptes de noyau à concurrence de moins de 5 % en poids et la surface vitreuse se composant de SiO₂, B₂O₃, K₂O, CaO, Al₂O₃ et ZnO, contenant les étapes consistant à :
- mettre les particules à noyaux en suspension dans un sol en utilisant une préparation de particules à noyaux
- lyophiliser la suspension pour obtenir un gel,
- épaissir le gel pour obtenir du verre,
**caractérisé en ce que** la préparation de particules à noyaux est constituée, à concurrence de 75 % en poids, par des particules dont la granulométrie se situe entre 0,5 et 15 µm.

15. Utilisation d'oxyde de zinc dans des couches vitreuses obtenues conformément au procédé sol/gel selon la revendication 14, pour augmenter l'aptitude à la liaison manifestée par la surface vitreuse pour des acides nucléiques.
